## Europäisches Patentamt

## European Patent Office

## Office européen des brevets

(19)

(11) Veröffentlichungsnummer: **0 062 904**
**B1**

(12) **EUROPÄISCHE PATENTSCHRIFT**

(45) Veröffentlichungstag der Patentschrift:
**05.02.86**

(21) Anmeldenummer: **82103031.9**

(22) Anmeldetag: **08.04.82**

(51) Int. Cl.⁴: **C 07 J 63/00, A 61 K 31/12**

(54) **Pharmazeutische Präparate enthaltend D-homo-4,17-Androstadien-3-on.**

(30) Priorität: **13.04.81 DE 3115995**

(43) Veröffentlichungstag der Anmeldung:
**20.10.82 Patentblatt 82/42**

(45) Bekanntmachung des Hinweises auf die Patenterteilung:
**05.02.86 Patentblatt 86/6**

(84) Benannte Vertragsstaaten:
**AT BE CH DE FR GB IT LI LU NL SE**

(56) Entgegenhaltungen:
**DE - A - 2 631 915**
**GB - A - 556 734**

(73) Patentinhaber: **SCHERING AKTIENGESELLSCHAFT**
**Berlin und Bergkamen,**
**Müllerstrasse 170/178 Postfach 65 03 11,**
**D-1000 Berlin 65 (DE)**

(72) Erfinder: **Laurent, Henry, Dr., Glambecker Weg 21,**
**D-1000 Berlin 28 (DE)**
Erfinder: **Schleusener, Annerose, Dr., Ilsensteinweg 1B,**
**D-1000 Berlin 38 (DE)**
Erfinder: **Albring, Manfred, Dr., Am Dachsbau 34A,**
**D-1000 Berlin 27 (DE)**
Erfinder: **Wiechert, Rudolf, Prof., Dr., Petzower**
**Strasse 8a, D-1000 Berlin 39 (DE)**

LIBER, STOCKHOLM 1986

## Beschreibung

### Pharmazeutische Präparate enthaltende D-homo-4,17-Androstadien-3-on

Die Erfindung betrifft pharmazeutische präparate, die durch einen Gehalt an 0,05 bis 5,0 Gewichtsprozent D-homo-4,17Androstadien-3-on gekennzeichnet sind.

4,16-Androstadien-3-on ist eine seit langem bekannte Verbindung uon der man weiß, daß sie bei der Metabolisierung anderer Steroide, wie zum Beispiel Testosteron (J. Biol. Chem. 236, 1961, 692 ) oder Dehydroepiandrosteron (Steroids 8, 1966, 511 ) gebildet wird. Trotzdem ist nicht bekannt, daß diese Substanz jemals eingehender pharmakologisch untersucht wurde, offensichtlich deshalb, weil diese Verbindung einen ausgeprägten 'Harngeruch' hat ( Helv. Chim. Acta 28, 1945, 618 ), der sie als Arzneimittelwirkstoff ungeeignet macht.

Von Derivaten des 4,16-Androstadien-3-ons ist bekannt, daß sie bei lokaler Applikation antiandrogen wirksam sind, (Deutsche Offenlegungsschrift DE-A 26 31 915 und südafrikanisches Patenet SA-A 78 01974). Aber diese Verbindungen haben den Nachteil, daß ihre Wirksamkeit relativ gering ist, oder daß sie auch noch erhebliche unerwünschte systemische antiandrogene Wirksamkeit entfalten. Strukturanaloge vorbekannte Verbindungen, denen diese Nachteile nicht anhaften, haben aber, soweit eigene Versuche erkennen lassen, ebenfalls einen intensiven 'Harngeruch', wie an späterer Stelle dargelegt wird.

In der britischen Patentschrift GB-A-556734 wird das D-homo-4,17Androstadien-3-on selbst erwähnt über seine pharmakologischen Eigenschaften wird aber nichts berichtet.

Es wurde nun gefunden, daß dieses D-homo-4,17-Androstadien-3-on eine ausgeprägte topische antiandrogene Wirksamkeit besitzt, darüberhinaus die Lipogenese stark hemmt, und zusätzlich noch den Vorzug besitzt, daß es systemisch unwirksam und völlig geruchsfrei ist, wie die nachfolgenden Testergebnisse zeigen.

Die antiandrogene Wirksamkeit wird wie folgt bestimmt:

Männliche, fertile Hamster im Gewicht von etwa 80 g werden kastriert und mit täglich 0,1 mg Testosteronpropionst subcutan substituiert. Das rechte Ohr wird zweimal täglich mit 0,01 ml einer 3%igen Lösung der Testsubstanz in Aceton oder Ethanol 3 Wochen lang behandelt. Bei der Kontrollgruppe wird das rechte Ohr nur mit 0,01 ml Lösungsmittel behandelt.

Am 22. Tag werden die Tiere mit Ether getötet, Prostata und Samenblase werden präpariert und gewogen. Aus den Ohren werden jeweils definierte Gewebeareale von 3×7 mm Kantenlänge ausgestanzt, diese werden histologisch weiterbehandelt und die Areale der Talgdrüsen gemessen. Durch Vergleich der Areale der ventralen Seite der Ohren der mit dem Antiandrogen behandelten Tiere mit den gleichen Bereichen der nur mit Lösungsmittel behandelten Tiere erhält man ein Maß für den lokalen antiandrogenen Effekt der Testsubstanz. Die gegenüber einer Kontrollgruppe beobachtete Reduktion des Prostatagewichts und Samenblasengewichts zeigt das Ausmaß der systemischen antiandrogenen Wirksamkeit der Testsubstanz.

Die Beeinflussung der Lipogenese durch die Testsubstanzen wird wie folgt ermittelt:

Männliche, fertile Hamster im Gewicht von etwa 80 bis 100 g werden kastriert und mit täglich 0,1 mg Testosteronpropionat subcutan substituiert. Das rechte Ohr jedes Versuchstieres wird zweimal täglich mit je 0,01 ml einer 1 %igen Lösung der Testsubstanz in Aceton ( beziehungsweise bei der Kontollgruppe nur mit 0,01 ml Lösungsmittel ) drei Wochen lang behsndelt. Dann werden die Tiere getötet, und aus dem behandelten rechten Ohr sowie dem unbehandelten linken Ohr jeweils ein definiertes Gewebeareal von 8 mm Durchmesser ausgestanzt.

Ventrale und dorsale Seite der Ausstanzungen werden entlang des Ohrknorpels voneinander getrennt, unverzüglich in Dulbecco's Modifikation of Eagle's Medium, dem 4 mMol Glutamin und 10 % Kälberserum zugesetzt war und das zur Vermeidung uon mikrobieller Kontamination. 100 IE/ml Pencillin, 100 µg/ml Streptomycin, 125 µg/ml Kanamycin, 25 IE/ml Nystatin und 10 µg/ml Gentamycin enthält, überführt und eine Stunde lang bei 37 °C inkubiert.

Danach bringt man die Ausstanzungen unter sterilen Bedingungen in frisches Kulturmedium, das 1 µCi/ml $C^{14}$ markiertes Natriumacetat enthält und inkubiert 4 Stunden lang bei 37°C. Anschließend gibt man die Proben in 2 ml einer Proteolyse-Lösung aus 0,05 Mol Tris-Puffer uom $p_H$ = 7,5, 0,01 mol Dinatriumethylendiamintetraessigsäure, 0,5 % Natriumdodecylsulfat und 0,1 % Proteinase K ( Firma E. Merck A.G. Darmstadt, Bundesrepublik Deutschland), und inkubiert 24 Stunden lang bei 37 °C.

Die so erhaltenen Proben werden einmal mit 5 ml Chloroform und nochmals mit 3 ml Chloroform extrahiert, die uereinigten Chloroformextrakte im Vakuum eingeengt und ihr Gehalt an radiomarkierten Lipiden im Szintillationszähler ermittelt. Die prozentuale Hemmung der Lipogenese der behandelten Kontrollgruppe wird durch Vergleich mit der nur mit Lösungsmittel behandelten Kontrollgruppe berechnet.

Die nachfolgende Tabelle zeigt die in diesen Tests erhaltenen Ergebnisse.

Zur Herstellung pharmazeutischer Präparate wird das D-homo4,17-Androstadien-3-on mit den üblichen Tägersubstanzen zu Lösungen, Gels, Salben, Pudern oder anderen Präparaten verarbeitet werden. Geeignete Trägersubstanzen sind zum Beispiel Wasser, Ethanol, Propanol, Glycerin, Methylcellulose, Hydroxypropylcellulose, Carboxypolymethylen usw.. Das Antiandrogen wird in den Präparaten in einer Konzentration von 0,05 bis 5,0 Gewichtsprozent, bezogen auf das Gesamtgewicht des Präparates uerwendet. Die Präparate können zur topischen 8ehandlung von Erkrankungen, wie Akne, Seborrhoe, Alopezie und Hirsutismus verwendet werden.

Das D-homo-4,17-Androstadien-3-on kann in der Weise hergestellt werden, daß man D-homo-5,17-Androstadien-3ß-ol unter gleichzeitiger Isomerisierung der 5(6)-Doppelbindung oxidiert, beispielsweise unter den 8edingungen der sogenannten Oppennauer-Oxidation ( Carl Djerassi: Steroid Reactions. Holden-Day Inc.,San Francisco, 1963, Seite 89 ff ).

## Patentanspruch

Pharmazeutische Präparate gekennzeichnet durch einen Gehalt an 0,05 bis 5,0 Gewichtsprozent D-homo-4,17Androstadien-3-on.

## Claim

Pharmaceutical compositions characterised in that they contain from 0.05 to 5 % by weight of D-homo-4,17-androstadien-3-one.

## Revendication

Compositions pharmaceutiques caractérisées en ce qu'elles contiennent de 0,05 à 5 % en poids de D-homo-androstadièns-4,17 one-3.

### Tabelle

| Nr. | Substanz | Flächenreduktion der Talgdrüsen | | Gewichtsreduktion | | Lipogenese | | Harngeruch |
|---|---|---|---|---|---|---|---|---|
| | | behandeltes Ohr | Kontralaterales Ohr | Prostata | Samenbl. | behandeltes Ohr | Kontralaterales Ohr | |
| 1 | 4,15-Östradien-3-on | 74 % | 44 % | 0 % | 0 % | -28% | -8 % | sehr stark |
| 2 | 18-Methyl-4,16-östradien-3-on | 62 % | 18 % | 15 % | 10 % | -37 % | -7 % | stark |
| 3 | D-homo-4,17-Androstadien-3-on | 67 % | 24 % | 0 % | 0 % | -30 % | -12 % | geruchlos |